# EUROPEAN PATENT APPLICATION

(11) **EP 1 174 115 A2**
(43) Date of publication of application: **23.01.2002**
(21) Application number: 01116900.0
(22) Date of filing: 11.07.2001
(51) Int. Cl.: A61K 7/13, A45D 19/00

(54) **System for colouring hair**

(30) Priority: 20.07.2000 IT MI001650
(71) Applicant: Müster e Dikson Service SpA, 20023 Cerro Maggiore (Milan) (IT)
(72) Inventor: Colombo, Franco, 20023 Cerro Maggiore (Milan) (IT)
(74) Representative: Di Iorio, Vincenzo, Dr. Ing.

(57) **Abstract**

Colour-system to enhance or activate shades, reflections and coverings of the hair colourings, comprising a group of specific ingredients to form a product which is offered in the form of a liquid measurable (dispensable) in calibrated drops, and is to be used in association with other products for the treatment and colouring of hair, to obtain precise and predetermined colour results according to tables established on the basis of specific laboratory experiments and use controls.

## Description

This invention concerns a preparation, composition, ingredient and the like, in the form of a drops measurable solution to be associated with conventional agents for hair treatment in order to obtain precise experimentally pre-established effects of exaltation, activation, shadingy, reflectiony, synergy and the like of the colouring obtainable by said conventional agent.

More particularly, the invention refers to a product having a physical fluid structure formed by specific ingredients, measurable in drops, associable (by addition) to conventional agents for hair treatment, with which it interacts also sinergically causing a real pluri-component system that causes, surprisingly, combined effects which are extremely efficient and precise for hair colouring, and are prefixed and encoded in tables established on the basis of specific experimentations.

Therefore the invention concerns a preparation in the liquid form that can be precisely dispensed in drops, forming with conventional treatment agents, specially for hair colouring, a system (that will be termed as "colour-system") having interactive adjustable and orientable functionality by means of the simple batching in drops of the combining preparation or ingredient according to the invention.

This invention includes also processes for preparing said colour-system generator or coadiuvant and for the oriented application of the system related to the hair treatment phases.

Cited Prior Art. European Patent Publication EP 0788831 A2 describes a system for the dispensing of oxygen sensitive hair dye, concentrates without the use of an inert gas, comprising a plurality of collapsible-bags having an oxygen barrier.

Said dye concentrates along with oxygen activating liquids being sufficient to produce an infinite number of hair dye formulations by raising exact charges from said bags each connected to a separate precision pump whose piston lift excursion may be selectively set to determine the volume of the dye charge.

US Patent No. 5.316.481 a hair dye selection and display shelf system substantially comprising: - a first and a second plurality of hair dye products arranged by dye color shade graduation respectively by duration of hair dye effect along shelf horizontal and vertical axis; - a hair dye shade selector; and digitized instructional means.

Just for fixing better and soon the ideas, it is convenient to make reference to one of the most used hair treatments, that is presently in continuously increasing diffusion and evolution, for hair tinctures or colouring.

As it is well-known, the colouring is made applying a product (in particular a tincture obtained by oxidation or a direct colouring matter) onto the hair with a more or less standard batching, e.g. between about 40 and 60 g/ml for getting a complete application.

Hair dyes are on the market which form a part of oxidation tinctures that are termed as "Correctors", which are used in addition to cream tinctures (and said correctors have a cream aspect and consistency) for changing the colour shade of tincture that is used; these "Correctors" are normally used in a quantity from 0,1% to 10% (and exceptionally in a quantity higher than this value) with respect to the base tincture, that must be "corrected".

The batching of these "Correctors" is made normally "at first sight", that is causing the output of the "Corrector", having a cream aspect, from the feeding pipe and estimating the ejected volume, or "the number of centimetres" (as suggested by the major part of the Manufacturers of hair colouring agents). Accordingly, the batching system is rather empirical, aleatory and therefore subject to wide result variation.

After all, the correctors have well-established limits, as they are suitable to apply correction actions only for the specific effect of the product (tincture by oxidation), to which it is associated.

On the contrary an object of the present invention is to provide a hair colouring system (in the following indicated simply as colour-system) of a practically universal type, that causes aimed effects in association with substantially all of agents or products for hair treatment (tinctures; various colouring matters, balsams, creams, shampoos, etc.).

Another object of basical importance of the invention is to get a colour-system in a morphological form that allows an easy and, above all, precise batching, on the basis of colouring results that are experimentally predetermined.

This characteristic is also substantially critical and positive also because the colour-system according to the invention must be considered as "universal" not only because it can be applied to almost the totality of conventional preparations involved in the general treatment and in particular in the hair colouring (by oxidation tinctures, direct colouring matters, etc.), but further, according to another aspect of the invention because it causes on said preparations a colouring action or effect that is a function, at a parity of basic preparation, of batching or measuring in drops of the generator of the colour-system.

In a very simple and practical and therefore advantageous and preferred embodiment, the colour-system according to the invention is not formed in the conventional half-solid or cream state, but on the contrary it is prepared in the fluid (liquid) state contained in an easy container provided with a dispenser.

Preferably the container is of the bottle type provided with closing cap, preferably threadable on the top of the same bottle, including, as drawing and batching member, a dropper.

After all, one of the more innovative aspects of the invention must be seen in the fact that it is now embodied an hair colouring system, measurable in drops, that allows to get prefixed results, according to operative tables set up in laboratory after numerous tests and verified by multiple applications in various hair, in order to obtain well-established, constant and sure results.

Therefore the hair colouring system according to the invention has the form of a solution measurable in drops and is differentiated substantially from the products that are actually commercially available.

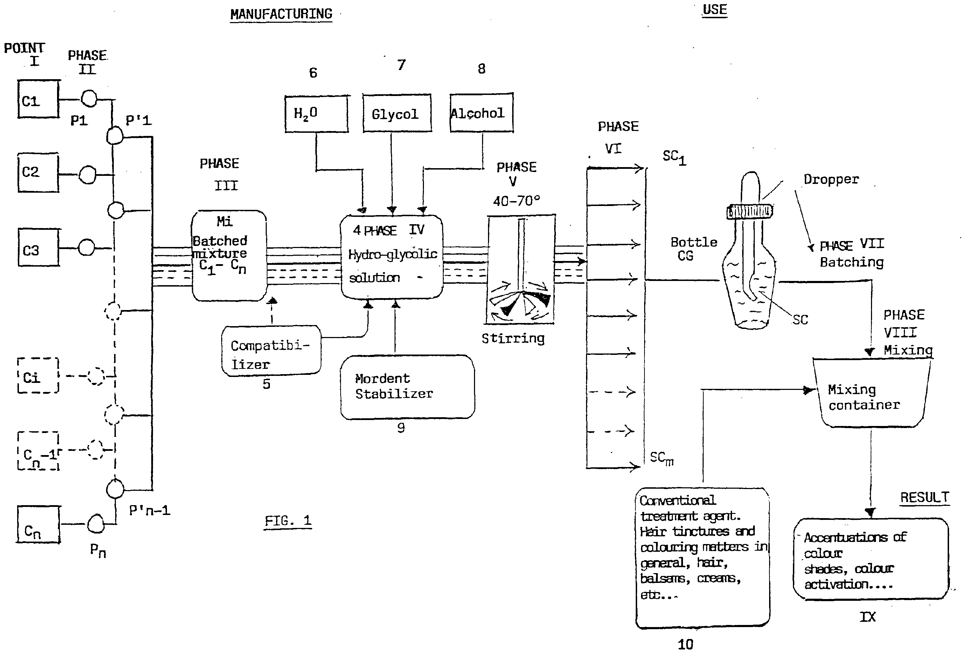

For fixing better the ideas (but without introducing limitations and bindings), the Figure 1 shows schematically a preferred embodiment of the process for the preparation of colour-systems (SC) according to the invention.

It is started generally from a series of colouring dyes from C1 to Cn (point 1) that, by weighting operations (P1-Pn), are singularly weighted and ratio referred the ones to the other ones according to specific formulations (phase II). In this manner there are prepared mixtures (Mi) made of many weighted and batched colouring matters in prefixed preparations according to the final colour-system SC that one wishes to obtain. Therefore it is operated with precise formulations, prefixed, set up after long laboratory experiments and verified by specific applications on hair and on many persons, in order to obtain precise and prefixed colouring results as a function of the amount of used and drop-batched colour-system.

The colours weighted in the relative, experimentally predetermined quantities shall form (in the phase III) the mixtures Mi that in the phase IV shall be added to a hydro-glycol/alcoholic solution (4) prepared in a mixing container, in which there are added: water (6), glycol (7) and/or alcohol (8), and further the mordant and the stabilizer (9). Moreover in the cases in which said mixture (Mi) contains colouring matters that are incompatible one with the other (e.g. acids and bases), it will be added a stabilizer-compatibilizer (5) to the same mixture during the phase III and/or to the solution 4 during the phase IV.

The solution IV formed in this manner (that can further contain other additives well-known per sé and especially to the mean skilled technical expert of this field, whereby their description can be omitted) is submitted to the thermal treatment at 40-70°C under stirring until a complete solubilization (phase V).

From this solution it will be obtained, during the phase VI, m colour-systems (SC1-SCm) according to the invention, that shall be each introduced in holders, preferably of the bottle type (VII) supplied with cap having a dropper (CG).

The application of each colour-system (SC), made by the user, shall occur with the maximum ease, precision, and efficiency, pouring the drops in number prefixed by the cases established and referred on the supplied reference tables, in a container for preparing the colouring mixture, in which it was preliminarly introduced a conventional treatment agent (10).

By mixing a general hair tincture with the colour-system (Sci) according to the invention, it will be obtained (in IX), according to the batching (drop number) of SCi, surprising effects of shades, accentuations and colour increments in the declared colour of the conventional treatment product.

In a very practical and advantageous embodiment for covering almost the complete totality of the exigencies, the colouring matters C1-Cn (according to Colour Index) were seleted from the group including:
Basic Yellow 57, Basic Brown 4, Basic Yellow 11, Disperse Violet 4, Disperse Red 15, Basic Red 2, Basic Red 76, Basic Violet 14, Basic Blue 99, Basic Blue 26, Basic Blue 7, HC Yellow No. 4, Disperse Blue 3, HC Red No. 3, HC Blue No. 2.

Obviously this number (n=15) and the colour types can be submitted to changes.

Preferably the solution ingredients are selected among Propylene Glycol, Poly-oxyethylen-glycol of the hydrogenated castor-oil, Metoxy-Isopropanol (acting also as mordant and stabilizer of the acid and basic colour combinations), Polysorbate 20, Nonixylol-9, Sorbithol, Glucose, Saccharose, Lactic acid (besides parfume in little quantities).

In an invention embodiment, with these starting colours and ingredients were prepared six colour-systems (SC1-SC6) according to the invention, catalogued as: straw, gold, copper, red, fuchsia and amaranth colours. Obviously this number is not binding and can be also changed.

Typically these colour-systems are used as coadivant, exalting, synergyzing agents and the like, and in addition, mainly to the following conventional treatment agents.
1) As addition to oxidation tinctures for getting an accentuation or a colour increment in the declared colour, and further an increase in the "covering". These tinctures can be in cream or in oily version or in gel version.
2) As addition to colouring shampoos in cream version, oily or gel version.
3) As addition to direct dyes for hair, not belonging to oxidation type, both in liquid and cream version, oily or gel version.
4) As addition to reflection agents for hair, in liquid, cream, oily or gel versions.
5) As addition to HYDROGEN PEROXIDE, OXIDATIVE EMULSIONS.
6) As addition to no colouring emulsions or creams, such as hair balsams or creams, for getting colouring or reflection effects.
7) As addition to shampoos and/or finishing products, for getting colourings or reflection effects in the hair.
8) As addition to COLOURING or DECOLORANT CREAMS for getting colouring or reflexing effects, according to cases.

The table I shows the results that can be obtained by the application of the six above mentioned colour-systems (column 1) in the doses (number of drops) referred in the columns from 2 to 5, for doses of 40-60 grams of conventional tincture.

**TABLE I**

| Colour-system (colour drops) | | | | |
|---|---|---|---|---|
| 1 | 2 | 3 | 4 | 5 |
| Colour-system (colour drops) | For very clear and extra-bleaching blonde hair | For mean and clear blonde hair | For dark blonde hair and clear-brown hair | For mean and dark brown hair |
| STRAW-COLOUR | | | | |
| * for supplying delicate Corn-Blonde shades to all colours of the Natural series, Gold and Ash colours, starting from very clear blonde colours until dark blonde and clear brown colour | 2-4 drops | 4-8 drops | 8-15 drops | / |
| GOLD COLOUR | | | | |
| * for getting or increasing Gold shades to all colours of the Natural series, Gold, Copper, Red, starting from the very clear blonde colour until the dark blonde and clear brown colour | 2-4 drops | 4-8 drops | 8-15 drops | / |
| COPPER COLOUR | | | | |
| * for Increasing lively Copper shades to all tincture colours, starting from clear and very clear colours (8-9) until the dark blonde and clear brown (with these darker colours, use the batching higher than 15 drops | 2-4 drops | 4-8 drops | 8-15 drops | / |
| Colour-system (colour drops) | For very clear and extra-bleaching blonde hair | For mean and clear blonde hair | For dark blonde hair and clear-brown hair | For mean and dark brown hair |
| RED COLOUR | Optional | | | |
| * for increasing the Red shades in fashion to all tincture colours, starting from colours having tonalities no. 8-9 (clear and very clear blonde colours) until the tonalities no. 5-6 (clear brown/dark blonde). For increasing the same colours of the Red series, with colours having tonalities no. 5-6, use 8-15 drops. | 2-4 drops | 4-8 drops | 8-15 drops | / |
| FUCHSIA COLOUR | | | | |
| * for supplying vivacious Red-Rubine shades to all tincture colours, starting from the colours of tonalities no. 7-8 (mean blonde and clear blonde colours) until dark colours, For increasing the same colours of the Red series. | Too Intense | 4-8 drops | 8-15 drops | 15-20 drops |
| AMARANTH COLOUR | | Facultative | | |
| * for supplying Red-Amaranth shades to colours ranging from dark Blonde to Brown (clear and dark) colour. For increasing the colours of the Red series. | Too intense | 4-8 drops | 8-15 drops | 15-20 drops |

### NOTES

- The use of more than 20 drops, in addition to mean, clear and very clear colour tinctures, impart very marked shades, where the prevailing colour is that of the used colour-system (SC). Therefore it will obtained a fantasy result and no more conforming to the tonality of the used tincture.
- For very clear and extra-bleaching blonde colours, use from 2 to 4 product drops for not diverging too much from the declared colour of used tincture (excepting that is wished a sharp increment of added colour).
- Fuchsia and Amaranth Colour-System can be used also for the black, for getting particular and delicate customised shades, using from 15 to 20 drops.
- The drops formed by dropper that can be used for SC according to the invention, can change from 0,015 to 0,150 g, preferably from 0,020 to 0,060 g.

Preferably it is used droppers gauged from 0,015 to 0,150, preferably from 0,020 to 0,060 grams/water.
- It can be used pump batchers (of mechanical or spray types) for single batchings e.g. from 0,150 to 5 g.

Obviously the use modalities are extremely simple:
- The hairdresser pours 40-60 g of tincture in the container foreseen for the mixing, he adds the oxidiser (H₂O₂ or oxidizing emulsion) and then the colour drops as in columns 2-5 of table 1 (or similar tables referred in the packages) for getting the shades declared in column 1, and then making the mixing and the application.

For simplifying the description and making easier the comprehension, the invention has been purposely described with particular reference to more simple embodiments and in particular in relation to the scheme of Figure 1 and to frequent cases of hair colouring indicated in table 1. It is evident that the invention is not limited to these fulfilment forms and can be subject to those modifications, variants, replacements, additions and the like that, being in the reach of the mean skilled technician, can be considered as naturally included in the scope and spirit of the invention, as described, for example, in the annexed claims.

## Claims

1. Colour-system for enhancing or activating shades, reflections, covering and general characteristics of the hair colouring, comprising a first ingredient or component including at least a treatment agent of said hair, **characterised in that** it includes at least a second additional ingredient in liquid form, that can be batched in gauged drops for getting precise and prefixed colour results in association with said first treatment agent.

2. Colour-system according to claim 1, **characterised in that** the liquid ingredient is added in drops to the starting colouring agents, in particular to oxidation tinctures, direct tinctures, reflecting agents, colouring shampoos and the like in cream version or in gel version, for getting precise, prefixed colour variations in said colouring starting agents.

3. Colour-system according to claim 1, **characterised in that** it is added in drops to uncolouring emulsions or creams, in particular to hair balsams or creams for getting hair reflections or colouring.

4. Colour-system according to claim 1, **characterised in that** it is added in drops to uncolouring shampoos and/or finishing products, for getting hair reflections or colourings.

5. Colour-system according to claim 1, **characterised in that** it is added in drops to oxidizing products, in particular hydrogen peroxide and oxydizing emulsions, for getting hair reflections or colouring.

6. Colour-system according to claim 1, **characterised in that** it is added in drops to general decolorant creams and decolorant agents, for getting hair colourings or reflections.

7. Colour-system according to at least one of preceding claims, **characterised in that** it is batched (dispensed) in drops from at least 1 to at least 25 drops in a sole or multiple application phase.

8. Colour-system according to claim 7, **characterised in that** the drops have a weight included between 0,015 and 0,150 preferably between 0,020 and 0,060 grams.

9. Colour-system according to claim 7, **characterised in that** the drops are formed by calibrated droppers from 0,015 to 0,150, preferably from 0,020 to 0,060 grams/water.

10. Colour-system according to at least one of claims 1 and 7-9, **characterised in that** it is used pump or spray batchers for mono-batchings from 0,150 to 5 grams.

11. Colour-system according to at least one of preceding claims, **characterised in that** it includes:
- at least a colour selected from the group, including basic dyes, acid dyes and dispersed dyes;
- at least a mordent and/or stabilizing agent;
- at least a solvent selected from the group including water, glycol, polyglycol, alcohol and related derivatives or precursors.

12. Colour-system according to claim 11, **characterised in that** the colouring matter is selected among: Basic Yellow 57, Basic Brown 4, Basic Yellow 11, Disperse Violet 4, Disperse Red 15, Basic Red 2, Basic Red 76, Basic Violet 14, Basic Blue 99, Basic Blue 26, Basic Blue 7, HC Yellow No. 4, Disperse Blue 3, HC Red No. 3, HC Blue No. 2.

13. Colour-system according to claim 11, **characterised in that** the mordent and/or the stabilizer is selected among lactic acid, Meloxy-isopropanol, poly-oxyetylenglycol of the hydrogenated castor oil.

14. Process for the preparation of colour-systems according to preceding claims, in which:
- a hydro-glycol and/or alcoholic solution is prepared;
- a mixture is prepared of dyes selected from the group of acid, basic and dispersed dyes, in number and in weight ratios according to formulation of the final product to be manufactured;
- to the hydroglycolic and/or alcoholic solution are added the mixture of weighted colouring matters and a stabilizer that keeps compatible among one another the basic and acid colouring matters possible present in said mixture;
- a mordent and/or stabilizing substance is added;
- the solution is heated between 40 and 70°C under stirring, until the complete solubilization of ingredients;
- and, according to the selected and weighted dyes hold in the mixture, colour-systems are prepared which are selected from the group including at least straw, gold, copper, red, fuchsia, amaranth, etc. colours.

15. Colour-systems and related processes substantially according to what described and represented.
